# EUROPEAN PATENT SPECIFICATION

(11) **EP 3 990 900 B1**
(45) Date of publication and mention of the grant of the patent: **20.08.2025**
(21) Application number: 20830928.6
(22) Date of filing: 10.06.2020
(51) Int. Cl.: C12Q 1/26, G01N 33/569, G01N 33/58, G01N 33/50, G01N 21/76

(54) **OXIDASE-BASED CHEMILUMINESCENCE ASSAY OF NEUTROPHIL LEUKOCYTES IN WHOLE BLOOD APPLICABLE TO POINT-OF-CARE (POC) MEASUREMENT OF ABSOLUTE NEUTROPHIL FUNCTION (ANF)**
AUF OXIDASE BASIERENDER CHEMILUMINESZENZTEST VON NEUTROPHILE LEUKOCYTEN IN VOLLBLUT ZUR POINT-OF-CARE (POC)-MESSUNG DER ABSOLUTEN NEUTROPHILENFUNKTION (ANF)
DOSAGE PAR CHIMIOLUMINESCENCE DE LEUCOCYTES NEUTROPHILES DANS LE SANG TOTAL, BASÉ SUR L'OXYDASE, APPLICABLE À LA MESURE AU CHEVET DU PATIENT DE LA FONCTION NEUTROPHILE ABSOLUE (ANF)

(30) Priority: 27.06.2019 US 201962867348 P
(43) Date of publication of application: 04.05.2022
(73) Proprietor: Binary LLC, Little Rock, AR 72217 (US)
(72) Inventor: ALLEN, Robert C., Little Rock, AR 72217 (US); ALLEN, John C., Little Rock, AR 72217 (US); STEPHENS JR., Jackson T., Little Rock, AR 72217 (US)
(74) Representative: K&L Gates LLP
(86) International application number: PCT/US2020/036998
(87) International publication number: WO 2020/263571

(56) References cited:
- WO-A1-2004/042395
- WO-A1-2018/060741
- WO-A1-91/06864
- US-A- 5 939 331
- US-A1- 2013 157 301
- US-A1- 2014 234 952
- US-B1- 6 383 479
- HASEGAWA H. ET AL.: "Analysis and assessment of the capacity of neutrophils to produce reactive oxygen species in a 96-well microplate format using lucigenin- and luminol-dependent chemiluminescence", J. IMMUNOL. METH., vol. 210, 15 December 1997 (1997-12-15), pages 1 - 10, XP004107722
- MINKENBERG I. ET AL.: "Lucigenin-dependent chemiluminescence as a new assay for NAD(P) H-oxidase activity in particulate fractions of human polymorphonuclear leukocytes", J. IMMUNOL. METH., vol. 71, 8 June 1984 (1984-06-08), pages 61 - 67, XP023992113
- HUBER N. ET AL.: "Measuring short-term stress in birds: Comparing different endpoints of the endocrine-immune interface", PHYSIOL. BEHAVIOR, vol. 182, 27 September 2017 (2017-09-27), pages 46 - 53, XP085244169
- STEVENS D.L. ET AL.: "Analysis of circulating phagocyte activity measured by whole blood luminescence: Correlations with clinical status", J. INFECT. DIS., vol. 170, no. 6, 1994, pages 1463 - 1472, XP009052553
- WITKO-SARSAT, V ET AL.: "Priming of Blood Neutrophils in Children with Cystic Fibrosis: Correlation between Functional and Phenotypic Expression of Opsonin Receptors before and after Platelet-Activating Factor Priming", THE JOURNAL OF INFECTIOUS DISEASES, vol. 179, January 1999 (1999-01-01), pages 151 - 162, XP055778738
- HAYANI, KC ET AL.: "Impaired Phagocyte Oxidative Capacity in Human Immunodeficiency Virus- Infected Children", THE JOURNAL OF INFECTIOUS DISEASES, vol. 179, March 1999 (1999-03-01), pages 584 - 589, XP055778742, DOI: 10.1086/314622

## Description

### TECHNICAL FIELD

Methods quantify the presence of phagocytic leukocytes, i.e., essentially neutrophils but also including monocytes and eosinophils, in blood and body fluids in a manner applicable to point-of-care diagnostic testing. Absolute neutrophil counts (ANC) can be performed to assess myelopoietic suppression, usually in association with chemotherapy or as a measure of inflammation. The methods disclosed herein can quantify phagocytes based on activation of respiratory burst metabolism and measuring the reductive dioxygenation of a chemiluminigenic probe.

### BACKGROUND

Automated blood analyzers are well-established instruments for the measurement of leukocytes, erythrocytes and platelets in whole blood. The leukocyte components of blood differ from erythrocytes and platelets morphologically and functionally. Impedance, light scatter and enzymatic and antigenic differences serve as the basis for counting and differentiating leukocytes by automated hematology. These instruments are highly complex and do not lend themselves to point-of-care (POC) assay development.

Activation of phagocyte metabolism, i.e., the "respiratory burst", is characterized by a large increase in glucose metabolism via the dehydrogenases of the hexose monophosphate shunt (pentose pathway) and a proportional increase in non-mitochondrial O₂ consumption (Sbarra and Karnovsky 1959). Both activities reflect the activation of NADPH oxidase (NADPH: O₂ oxidoreductase), an enzyme common to blood phagocytes (Rossi, Romeo et al. 1972). Activation of phagocyte NADPH oxidase drives combustive dioxygenation reactions that yield a native chemiluminescence as an energy product (Allen et al 1972). Native leukocyte chemiluminescence is O₂-dependent and directly proportional to hexose monophosphate shunt dehydrogenase activity. NADPH oxidase activation results in the generation of HO₂, O₂⁻ and H₂O₂. The H₂O₂ generated serves as substrate for MPO (H₂O₂:halide oxidoreductase) oxidation of chloride to hypochlorite (OCl⁻), and secondary chemical reaction with an addition H₂O₂ yielding singlet molecular oxygen (¹O₂*) (Allen, Yevich et al. 1974, Allen 1979). The halide-dependent haloperoxidase activity of isolated MPO also yield native chemiluminescence as an energy product (Allen 1975, Allen 1975).

Chemiluminescence quantum yield, i.e., the ratio of photons emitted per oxygenation event, is dependent on the type and quantity of oxygenating agent generated, and on the nature and quantum efficiency of the substrate oxygenated. Oxygenation of native substrates is associated with a relatively low chemiluminescence quantum yield and this yield varies with the nature of the substrate oxygenated. Introducing a high quantum yield chemiluminigenic substrate (CLS) overcomes the problems of sensitivity and substrate variability. Addition of cyclic hydrazides, e.g., luminol, increase the yield of phagocyte luminescence by greater than a thousand-fold (Allen and Loose 1976). Acridinium salts such as lucigenin also greatly increase leukocyte luminescence yield (Allen 1981, Allen 1982).

Cyclic hydrazide and acridinium chemiluminescence can be chemically elicited by exposure to H₂O₂ under alkaline conditions (Albrecht 1928, Totter 1964). However, these substrates do not yield CL in mildly acidic-to-neutral pH conditions of physiologic milieux. Luminol- and lucigenin-dependent phagocyte CL activities measure different oxygenation pathways (Allen 1982, Allen 1986). Luminol CL results from dioxygenations generating electronically excited aminophthalate, i.e., luminol + O₂ → aminophthalate + N₂ *+ photon.* In phagocytic leukocytes, such activity is strongly associated MPO. Luminol CL results from non-reductive, simple dioxygenation. A small luminol CL is observed in MPO-negative leukocytes, but MPO-positive leukocytes yield more than a hundredfold greater luminescence (Allen 1986, Merrill, Bretthauer et al. 1996, Allen 2019).

Phagocyte NADPH oxidase catalyzes the univalent reduction of O₂ to HO₂. In neutral milieu, HO₂ dissociates yielding O₂⁻ and H⁺, O₂⁻ and HO₂ disproportionate yielding H₂O₂. Under acid to neutral conditions, the divalent cationic lucigenin (N,N'-dimethyl-9,9' biacridinium and bis-*N*-methylacridinium) can undergo one electron reduction yielding the monovalent cation radical, i.e., lucigenin⁺⁺ + *e⁻* → lucigenin⁺, and this radical can react with O₂⁻ to yield a dioxetane intermediate, and ultimately, two N-methylacridone and a photon, i.e., lucigenin⁺ + O₂⁻ → lucigenin-O₂ → 2 N-methylacridone *+ photon* (Allen 1981, Allen 2019).

### SUMMARY

The present inventors surprisingly found that complex instrumentation required for impedance and flow cytometric measurements of neutrophils can be obviated by using a chemiluminescence approach to directly measure the functional activity of stimulated neutrophils present in diluted whole blood. The neutrophils per volume of whole blood are determined by measuring stimulated NADPH oxidase-dependent reductive dioxygenation of lucigenin as a chemiluminigenic substrate. Introducing a stimulus, such as PMA, that is optimal for activation of neutrophil NADPH oxidase results in generation of reductive dioxygenating activity.

The resulting reductive dioxygenation of lucigenin yields CL that can be quantified by measuring the light emitted using a luminometer. This luminescence is proportional to the metabolic activity of the neutrophils per volume of whole blood tested. This absolute neutrophil function (ANF) assay is proportional to the absolute neutrophil count (ANC) and is applicable for assessing the clinical state of a patient with regard to inflammation/infection or chemotherapy related bone marrow suppression. Such function-based analysis provides an alternative and arguably superior assay compared to the conventional ANC. The technical requirements of the ANF approach are applicable to point-of-care testing. As described in the Examples herein, the ANF assay allows quantitative assessment of neutrophil number (ANC) and function in blood during the initial sixteen-hour interval post venipuncture.

Additional features and advantages are described in, and will be apparent from, the following Detailed Description and the Figures.

### BRIEF DESCRIPTION OF DRAWINGS

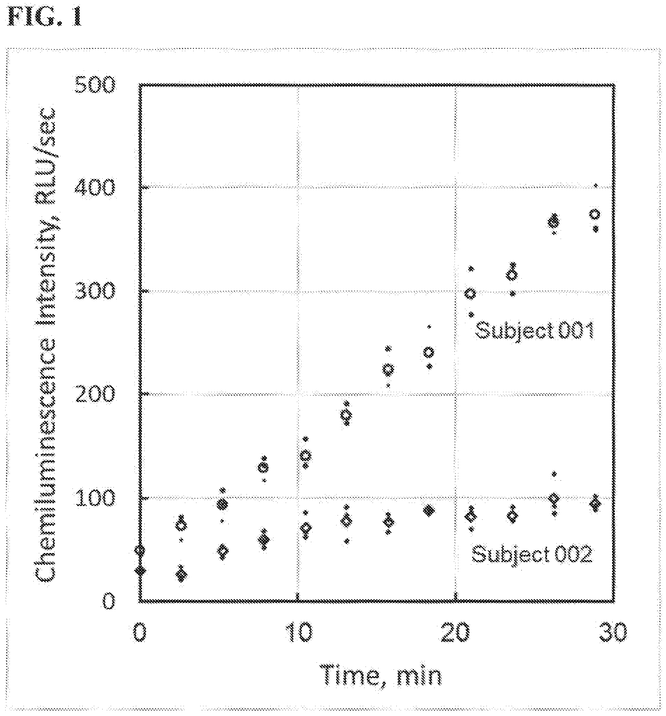

**FIG. 1** shows chemiluminescence intensity (velocity) in the experimental example disclosed herein, expressed as relative light units per second (RLU/sec), plotted against time expressed in minutes. A 0.25 µL equivalent volume of blood was tested for the first two Subjects enrolled, Subjects 001 (circles) and 002 (diamonds). Activation of CL occurred on mixing of an equivalent of 0.25 µL blood with 0.5 nmol PMA in a balanced-salt medium containing lucigenin (N,N'-dimethyl-9,9'-biacridinium dinitrate) as the chemiluminigenic probe. Measurements were made in triplicate and the average velocity is shown by the larger open marks with the standard deviation indicated by the small dots).

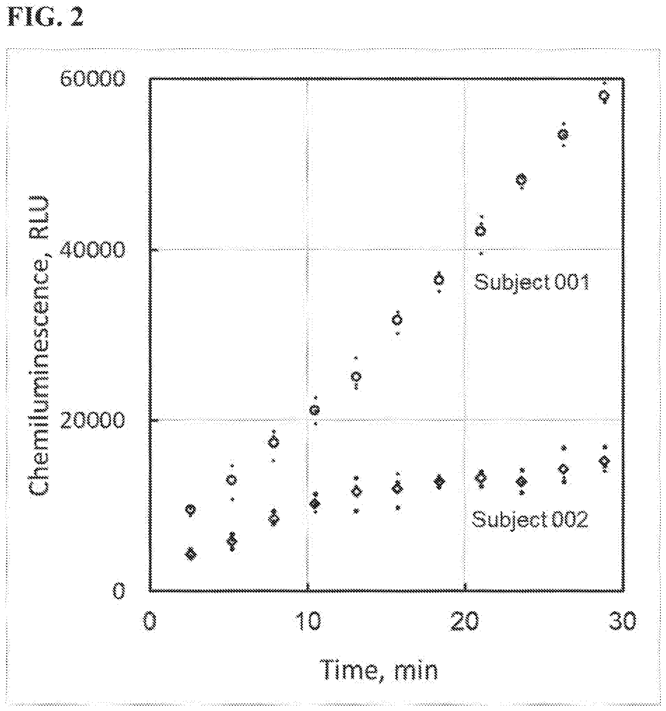

**FIG. 2** shows integral chemiluminescence in the experimental example disclosed herein, expressed as the accumulated relative light units (RLU) plotted over time expressed in minutes. The integral CL measurements (RLU) are calculated from the intensity measurements shown in **FIG. 1****.**

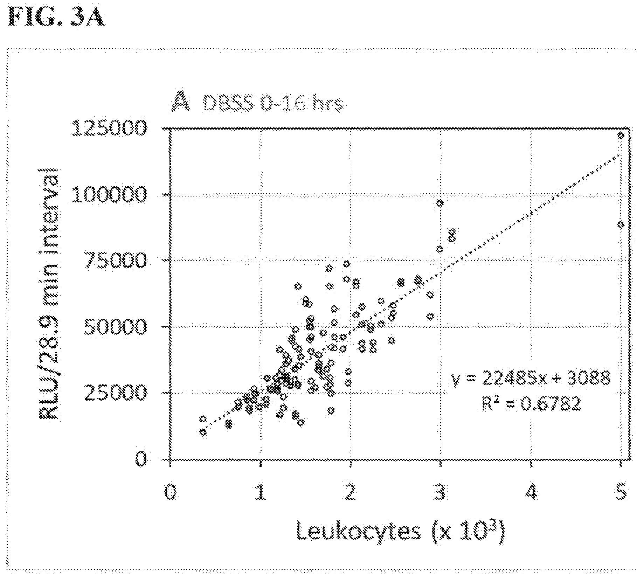

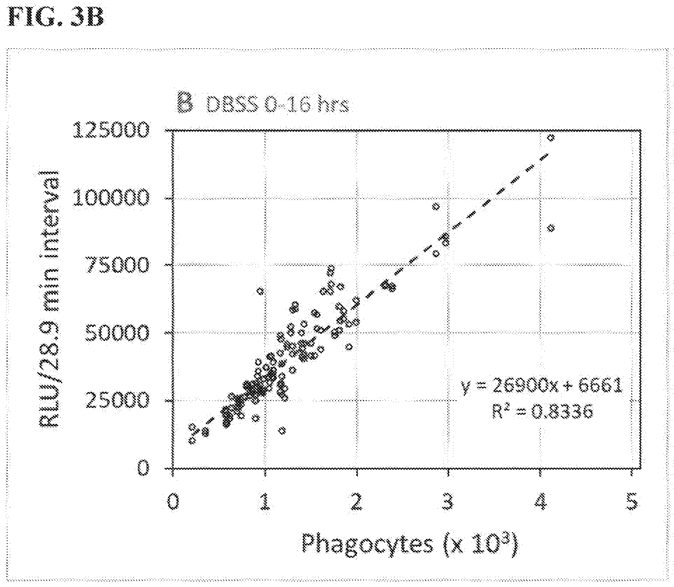

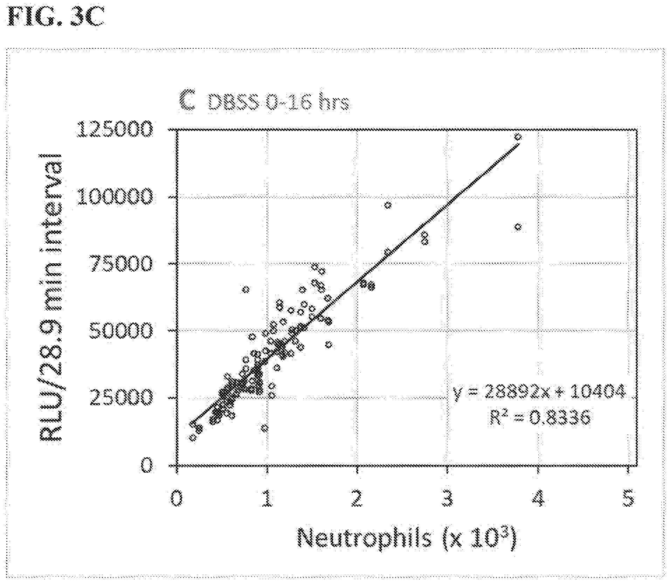

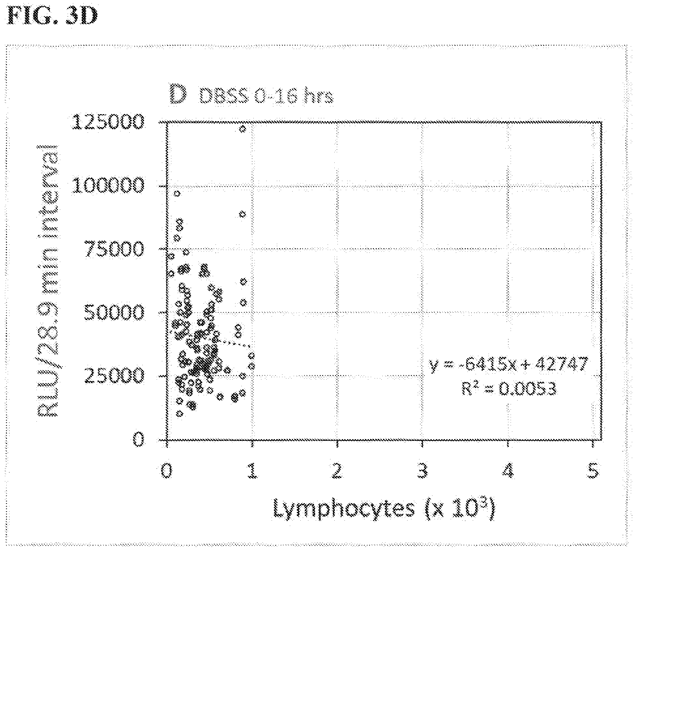

**FIGS. 3A****, B, C** and **D** are plots of integral lucigenin-dependent CL activities versus leukocyte, phagocyte (neutrophils, monocytes and eosinophils), neutrophils, and lymphocyte counts, respectively, in the experimental example disclosed herein. The abscissa describes the cell count per 0.25 µL blood plotted against oxidase-dependent reductive dioxygenation activity measured as PMA-stimulated lucigenin CL. Linear regression analyses with coefficients of determination (R²' s) are shown for each plot. The leukocyte counts were per 0.25 µL of blood tested. DBSS indicates that the medium was N,N'-dimethyl-9,9'-biacridinium dinitrate (lucigenin) balanced salt solution. The post-venipuncture age of the blood ranged from 1 to 16 hours.

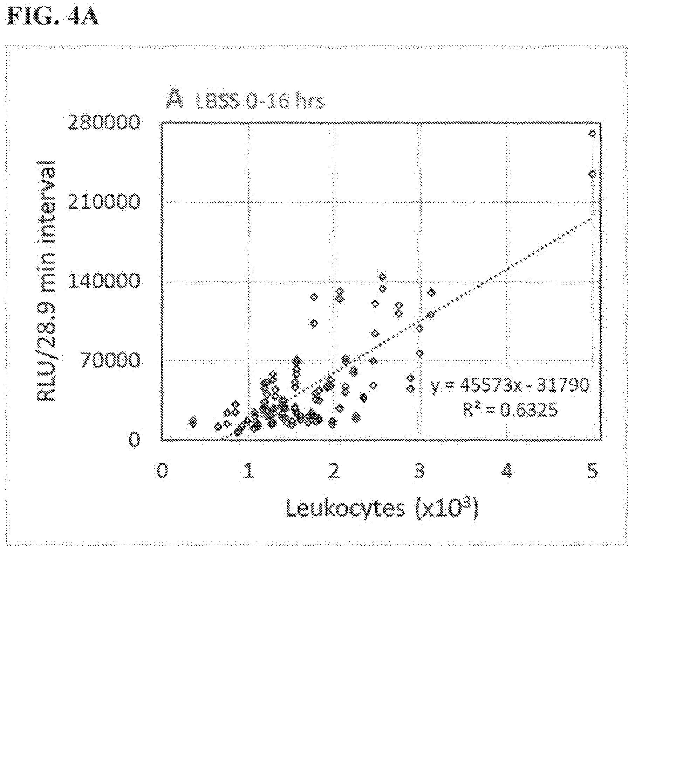

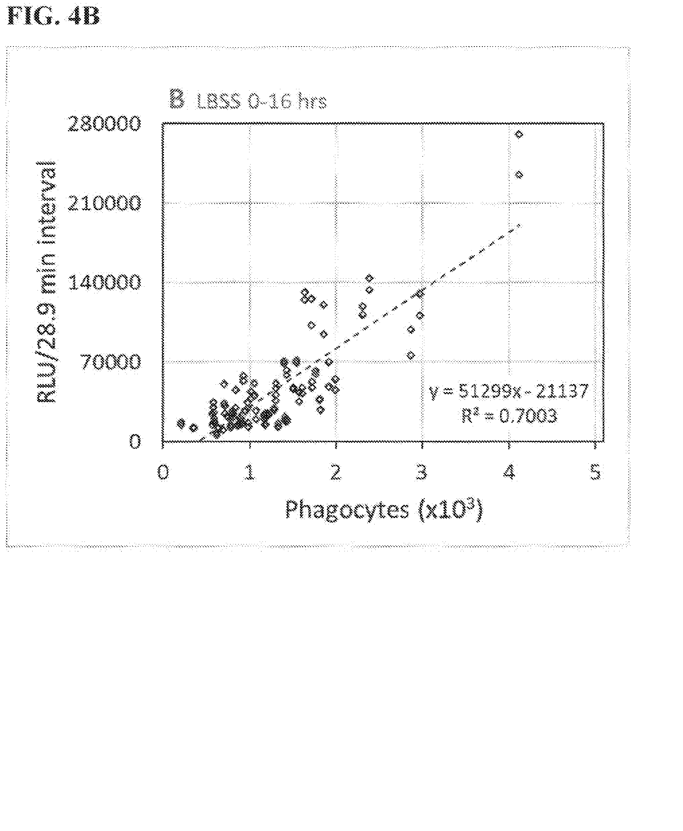

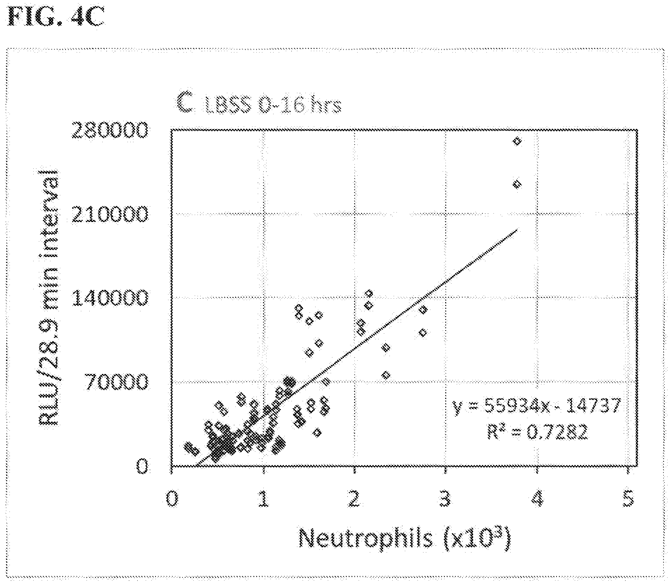

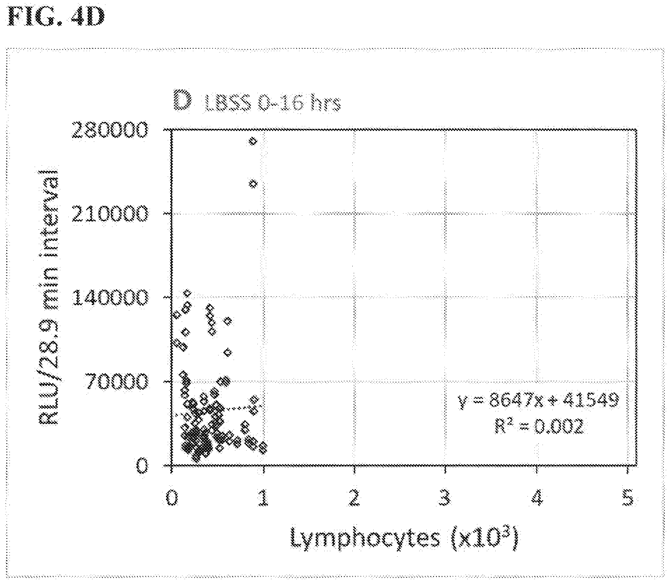

**FIGS. 4** **A, B, C** and **D** are plots of integral luminol-dependent CL activities versus leukocyte, phagocyte (neutrophils, monocytes and eosinophils), neutrophils, and lymphocyte counts, respectively, in the experimental example disclosed herein. The abscissa describes the cell count per 0.25 µL blood; the ordinate describes the oxidase-driven MPO-dependent (non-reductive) dioxygenation activity measured as PMA-stimulated luminol CL. Other than the chemiluminigenic probe, i.e., LBSS (luminol balanced salt solution), the conditions were as described for **FIGS. 3A-D.**

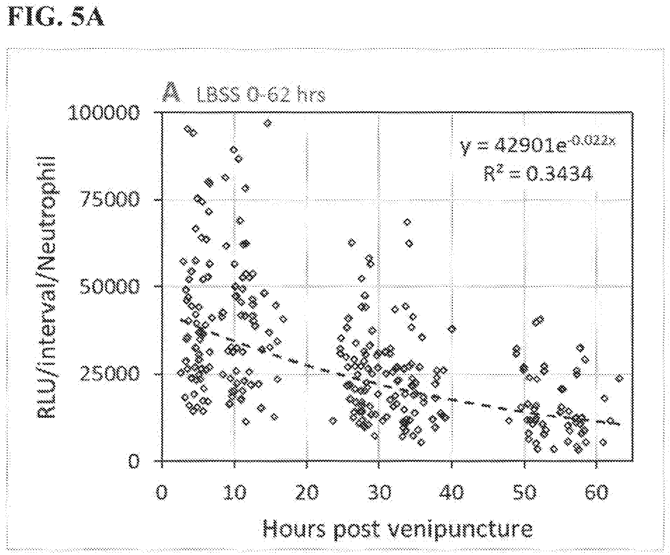

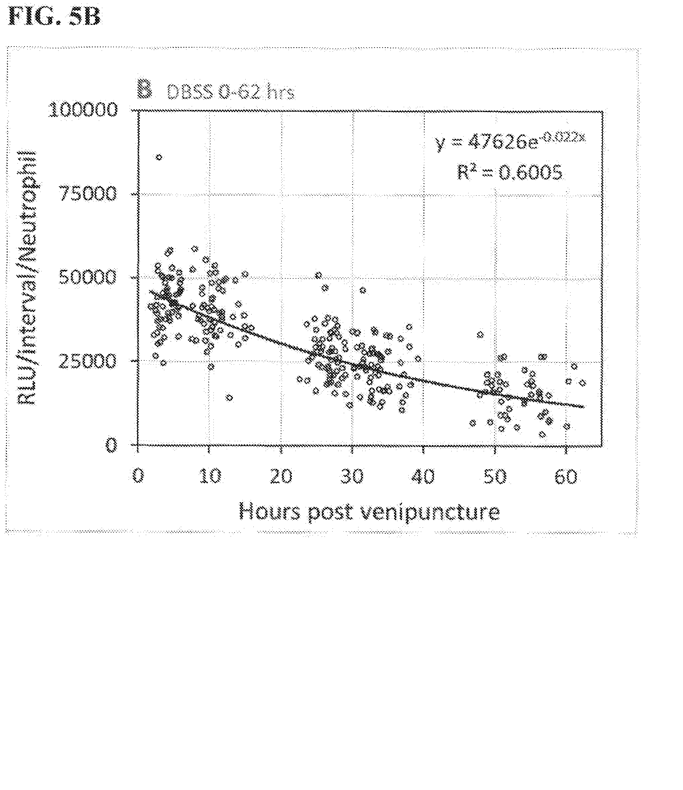

**FIGS. 5A** and **B** **are** CL activity plots from the experimental example disclosed herein. **FIG. 5A** is a plot of integral luminol-dependent CL activities per neutrophil versus the post-venipuncture age of the blood in hours. **FIG. 5B** is a plot of integral lucigenin-dependent CL activities per neutrophil versus the post-venipuncture age of the blood in hours. The testing interval for integration was 28.9 minutes.

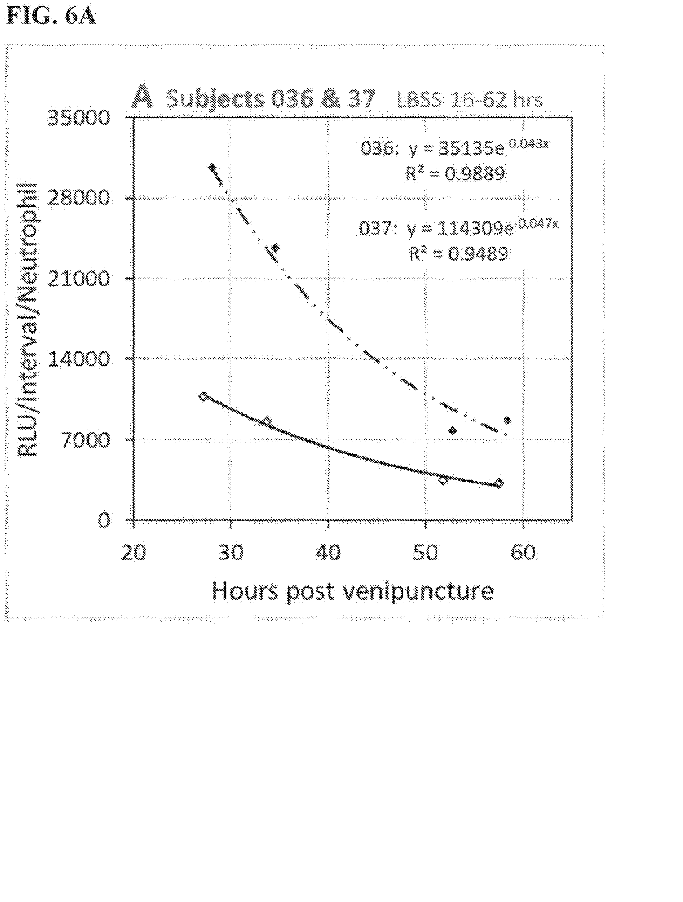

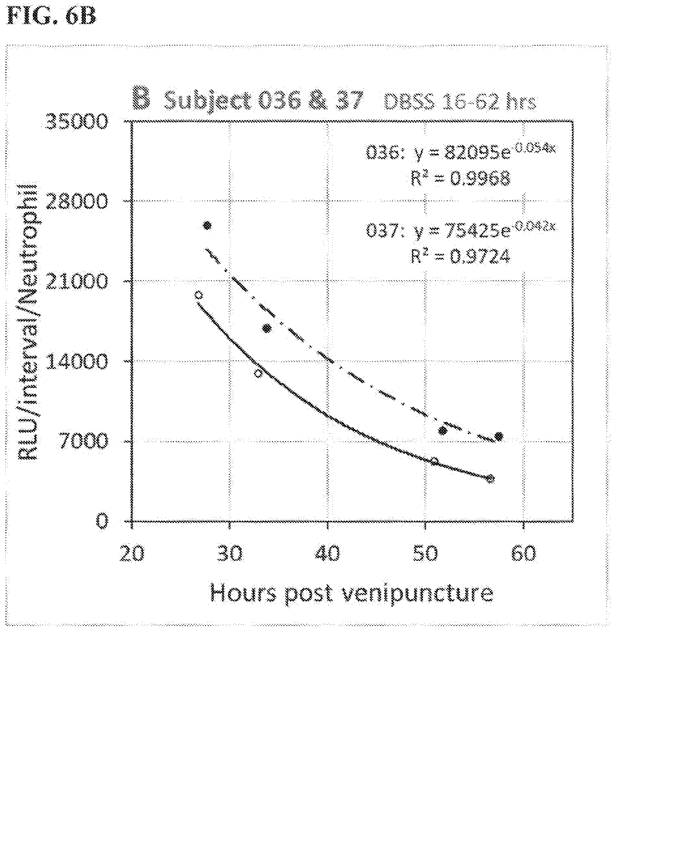

**FIGS. 6A** and **B** are CL activity plots from the experimental example disclosed herein. **FIG. 6A** is a plot of integral luminol-dependent CL activities per neutrophil versus the post-venipuncture age of blood for Subjects 036 and 037. **FIG. 6B** is a plot of integral lucigenin-dependent CL activities per neutrophil versus the post-venipuncture age of blood for Subjects 036 and 037. The testing internal for integration was 28.9 minutes.

### DETAILED DESCRIPTION

### General Embodiments

The present disclosure provides a method for estimating a number of phagocytes in a body fluid of an animal, the method comprising: stimulating NADPH oxidase activity of the phagocytes; and quantifying a resulting reductive dioxygenation of a chemiluminigenic substrate by an emitted chemiluminescence of the chemiluminigenic substrate using an instrument capable of measuring light.

In an embodiment, the NADPH oxidase activity of the phagocytes is stimulated by an immunologic or chemical capable of activating a respiratory burst by the phagocytes.

In an embodiment, the NADPH oxidase activity of the phagocytes is stimulated by a stimulus in solution or coated to a surface contacted by the phagocytes. The stimulus can be phorbol myristate acetate (PMA).

In an embodiment, the animal is a human.

The body fluid is whole blood.

The phagocytes are neutrophil leukocytes.

The chemiluminigenic substrate is lucigenin (N,N'-dimethyl-9,9'-biacridinium dinitrate). The lucigenin can be in solution or coated to a surface contacted by the phagocytes.

The method comprises diluting the whole blood 1 to 1200 to diminish erythrocyte absorbance of chemiluminescence.

In an embodiment, the method comprises using a lectin to aggregate or remove erythrocytes from the body fluid to facilitate chemiluminescence detection.

The emitted chemiluminescence is measured by a portable or hand-held luminometer. In an embodiment, the components are prefabricated to facilitate point-of-care testing.

The method further comprises using the estimated number of neutrophils to determine an absolute neutrophil count (ANC) and further comprises using the ANC to assess myelopoietic suppression in the animal, e.g., myelopoietic suppression is associated with chemotherapy or a measure of inflammation or infection. In this regard, inflammation or infection typically increases myelopoietic activity, but neutrophil consumption in response to infection can actually decrease the neutrophil count. The invention is defined by the appended claims.

The present disclosure also describes a method for estimating myelopoiesis stimulation is provided. The method comprises: measuring non-reductive dioxygenation-driven myeloperoxidase (luminol CL) activity and reductive dioxygenation (lucigenin CL) activity of chemically-activated blood neutrophils of an animal using an instrument capable of measuring light; and calculating a ratio of the luminol CL activity to the lucigenin CL activity.

### Preferred Embodiments

The absolute neutrophil function (ANF) assay disclosed herein includes a sensitive chemiluminigenic probe method for determination of the number of functional phagocytes, i.e., neutrophil leukocytes with minor contributions from monocytes and eosinophils, in diluted whole blood. Specifically, phagocyte function is quantified by introducing lucigenin as a chemiluminigenic probe and measuring the chemiluminescence product of stimulated NADPH oxidase reductive dioxygenation activity.

The method requires less than a drop of whole blood. A small volume of the specimen is diluted in a balanced salt solution. The diluted specimen is introduced into a milieu containing a chemical stimulant, such as phorbol 12-myristate 13-acetate (PMA), capable of activating phagocyte NADPH oxidase-dependent respiratory burst metabolism, and a chemiluminigenic probe susceptible to reductive dioxygenation, i.e., lucigenin (N,N'-dimethyl-9,9'-biacridinium dinitrate). Such contact activates phagocyte NADPH oxidase-dependent reductive dioxygenation of lucigenin yielding chemiluminescence that can be detected and quantified by luminometry. Chemiluminigenic probes such as luminol measure phagocyte non-reductive (simple) dioxygenation reactions, especially those catalyzed by myeloperoxidase (MPO).

Under normal myelopoietic conditions, measurement of non-reductive dioxygenation approximates the number of neutrophils present. Inflammatory stimulation and g-CSF treatment expand the promyelocytic pool of the bone marrow and can result in a several-fold increase in MPO content per neutrophils. Non-reductive dioxygenation activity reflects MPO content per phagocyte, and reflects the specific MPO content per phagocyte. NADPH oxidase-dependent reductive dioxygenation of lucigenin is not dependent on MPO, and consequently, is directly proportional to the number of functional phagocytes in the specimen. The lucigenin-based ANF system quantifies phagocyte presence by measuring stimulated phagocyte reductive dioxygenation. In the absence of genetic, e.g., chronic granulomatous disease, or an acquired neutrophil abnormality, lucigenin-dependent measurement of NADPH oxidase reductive dioxygenation activity is MPO-independent and closely approximates the neutrophil count of the specimen. The ANC determines the number of neutrophils present in a specimen. The ANF assay provides more clinically relevant information based on quantifying the functional presence of neutrophils in a specimen. The ANF assay is demonstrated to quantitatively reflect the whole blood ANC during the initial sixteen-hour post venipuncture interval, and is applicable to point-of-care testing.

Although NADPH oxidase activity per phagocyte is relatively constant, myeloperoxidase (MPO) activity per phagocyte varies with the state of myelopoietic stimulation. MPO and cationic proteases are lysosomal enzymes synthesized in the promyelocytic phase of development and stored in the azurophilic granules of neutrophils (Bainton 1999). The concentration of MPO per phagocyte is dependent on the degree of myelopoietic stimulation, i.e., activation by colony stimulating factors (physiologic or recombinant G-CSF or GM-CSF) and on the number of mitotic divisions during the myelocytic phase of neutrophil development (Allen, Stevens et al. 1997). For example, each division in the myelocytic phase dilutes the myeloperoxidase per neutrophil by one half. Components of NADPH oxidase are synthesized in the myelocytic phase of development, and required for respiratory burst metabolism. Consequently, the NADPH oxidase activity per phagocyte is relatively constant with regard to variation in myelopoietic activity. The specific NADPH oxidase activity per phagocyte remains relatively constant in various conditions of inflammation and myelopoietic stimulation or suppression.

As such, stimulated oxidase activity closely approximates the phagocyte count, especially the neutrophil count. Measurement of such activity closely approximates the absolute neutrophil count per volume of blood or body fluid. Introduction of lucigenin as chemiluminigenic probe allows sensitive quantification of phagocyte NADPH oxidase reductive dioxygenation activity (Allen 1981, Allen 1982, Allen 1986). A simple or non-reductive dioxygenation is a reaction where O₂ is incorporated in a substrate, e.g., luminol + O₂ → aminophthalate + N₂ + photon (Allen and Loose 1976). A reductive dioxygenation is a reaction where O₂ plus two reducing equivalents (2 electrons + 2 protons) is incorporated, e.g., lucigenin + 2 electrons +2 protons + O₂ → 2 N-methyl acridone + photon.

NADPH oxidase dependent reductive dioxygenation of lucigenin provides a measurement of absolute neutrophil function (ANF) that closely approximates the absolute neutrophil count (ANC). In addition to providing information consistent with an ANC, lucigenin chemiluminescence (CL) measurement of stimulated neutrophil oxidase activity provides useful clinical information and can be applied to point-of-care (POC) testing using a hand-held luminometer.

Activity-based measurement of neutrophils offers additional information. The ANC quantifies the physical presence of neutrophils, but does not quantify neutrophil function. As such, conditions associated with impaired phagocyte function, e.g., chronic granulomatous disease or any toxic effect of treatment on neutrophil function, are not detected. Functional measurement of the respiratory burst activity that drives reductive dioxygenation activity quantifies neutrophil microbicidal capacity. By analogy, the antigenic detection of an enzyme does not provide information with regard to its function. An enzyme may be antigenically present, but non-functional. Functional measurement of the enzyme provides more complete and clinically useful information; i.e., the enzyme is present and functional.

When stimulated neutrophils are quantified by measuring reductive dioxygenation of lucigenin, the CL response correlates with the neutrophil count. When stimulated neutrophils are quantified by measuring non-reductive dioxygenation of luminol, the CL response shows less correlation with the neutrophil count. Chemiluminigenic probes such as luminol measure non-reductive dioxygenation or dioxygenation activity, especially those catalyzed by MPO (Allen 2019). Under normal myelopoietic conditions, the MPO content per neutrophil is stable, and luminol dioxygenation activity roughly approximates the number of neutrophils present. However, in clinical conditions associated with increased myelopoietic activity, e.g., g-CSF treatment and inflammatory stimulation, the promyelocytic pool of the bone marrow is expanded, and there are fewer mitotic divisions in the myelocytic pool of the marrow. MPO is synthesized only during the promyelocytic stage of development. Inflammatory stimulation or therapeutic treatment with G-CSF stimulates and expands the promyelocytic pool and decreases the number of divisions in the myelocytic pool. Consequently, the azurophilic granules containing MPO are not diluted by mitoses in the myelocytic phase of development. Neutrophils synthesized under such stimulated myelopoietic conditions show several-fold increases in MPO per neutrophils (Allen et al 1997).

The ANF method disclosed herein is highly sensitive and can be performed on less than a microliter of diluted whole blood. As the following examples demonstrate, the ANF chemiluminigenic technique for quantifying phagocytes in blood or body fluids provides the functional equivalent of an ANC. This lucigenin CL method is technically flexible and is applicable to point-of-care testing (POCT) using a portable or handheld luminometer for measurement. The acceptance and demand for POCT continues to increase (Asha, Chan et al. 2013, Schilling 2014). No handheld POCT method is available for ANC (POCT).

### Examples

The following non-limiting examples provide scientific data supporting the concepts disclosed herein.

Human blood specimens tested were provided by a local clinical testing laboratory. De-identified blood specimens were obtained with time of venipuncture, age (in years), sex of the Subject, and the automated hematology analyzer (Advia 120, Siemens AG) printout. The reason for ordering the complete blood count and information on the Subject's medical condition were unknown.

The blood specimens were maintained at ambient temperature (22 ± 8 C°) until tested. Each of the 58 blood-specimens were tested in triplicate. The average (mean) post-venipuncture age of the blood with standard deviation (SD) at initial testing was 4.1 ± 1.1 hrs and the median post-venipuncture age was 4.1 hrs.

The specimens were tested again about 6 hours later; the average post-venipuncture age with a SD was 10.8 ± 2.0 hrs and the median was 10.4 hrs. The blood was also tested at later times post-venipuncture in an attempt to establish a limit of acceptability with regard to the post-venipuncture age of the specimen.

Acceptably reproducible neutrophil functional activities were obtained over the initial 16 hrs post-venipuncture period, and as such, the combined venipuncture average (mean) with SD of 7.4 hrs ± 3.8 and a median of 10.4 hrs was used for analysis. The Subjects included 23 males and 35 females. The age range was 21 to 99 years with an average age of 69.4 and an SD of 17.5 and a median age of 70.5 years. A total of 116 measurements were taken.

The media employed included diluting media (DM), luminol balanced salt solution (LBSS) and lucigenin (dimethylbiacridinium dinitrate) balanced salt solution (DBSS). DM contained: 5 mM 3-(N-morpholine) propanosulfonate (MOPS)-buffered balanced salt solution (139 mEq/L Na⁺, 5.0 mEq/L K⁺, 132 mEq/L Cl⁻, 0.8 mM HₙPO₄; pH 7.2; 290 ± 5 mOsmol/kg and endotoxin <0.06 endotoxin units (EU)/mL. LBSS contained: 5 mM MOPS-buffered salt solution containing 139 mEq/L Na⁺, 5.0 mEq/L K⁺, 1.3 mM Ca²⁺, 0.9 mM Mg²⁺, 142 mEq/L Cl⁻, 0.8 mM HₙPO₄, plus 0.15 mM luminol (5-amino-2,3-dihydro-1,4-phthalazinedione) and 5.5 mM D-glucose; pH 7.2; 290 ± 5 mOsmol/kg and endotoxin <0.06 EU/mL. DBSS contained: 5 mM MOPS-buffered salt solution containing 139 mEq/L Na⁺, 5.0 mEq/L K⁺, 1.3 mM Ca²⁺, 0.9 mM Mg²⁺, 142 mEq/L Cl⁻, 0.8 mM HₙPO₄, plus 0.2 mM lucigenin (*N,N'*-dimethyl-9,9'-biacridinium dinitrate; aka bis-N-methylacridinium nitrate) and 5.5 mM D-glucose; pH 7.2; 290 ± 5 mOsmol/kg and endotoxin <0.06 EU/mL.

**FIG. 1** shows the plot of chemiluminescence intensity (velocity) expressed as relative light units (RLU/sec) measured over a 29 min interval plotted against the time interval of testing for first two subjects tested. The potassium ethylenediaminetetraacetate (K₃EDTA)-anticoagulated whole blood specimens were initially diluted with DM and tested at a final dilution of 1 to 1200. Testing was performed in triplicate. A 0.25 µL equivalent volume of blood was used per test. Activation of phagocyte respiratory burst metabolism was initiated on addition of the diluted blood to a microplate well coated with 0.5 nanomole (nmol) phorbol 12-myristate 13-acetate (PMA), i.e., stimulus, and containing balanced salt solution with a chemiluminigenic probe, i.e., lucigenin or luminol. The final volume per well was 300 µL.

Measurements were taken at 2 min 37 sec intervals using an Orion II microplate luminometer (Titertek Berthold) at a temperature of 37°C. For example, the absolute leukocyte count (white blood count; WBC) for Subjects 001 and 002 were 2475/0.25 µL and 350/0.25 µL blood, respectively, and the absolute neutrophil counts (ANC) for Subjects 001 and 002 were 1,525/0.25 µL and 178/0.25 µL, respectively. The post-venipuncture age of the blood specimens for Subjects 001 and 002 were 5 hrs 40 min and 2 hrs 49 min, respectively.

For each Subject, twelve chemiluminescence (CL) intensity measurements were taken over a 28.9 min interval. As depicted in **FIG. 1****,** CL activity is measured as intensity, i.e., a velocity, and expressed as relative light units per second (RLU/sec). As such, the plotted data illustrate the change in CL intensity (velocity) with respect to time. It is important to appreciate that CL measurements are velocity (rate) measurements whereas most techniques measure accumulation of product or depletion of substrate, i.e., integral values.

For some comparisons, it may be appropriate to express CL as an accumulated RLU, i.e., integral or summation value, over a selected time interval of testing. The CL velocity (RLU/sec) values can be converted to integral CL expressed as RLU values by summation of the area under the RLU/sec plots over the interval of testing.

**FIG. 2** presents the CL data of **FIG. 1** expressed as the total or integral RLU's accumulated during the time interval of measurement. Integral expression of CL may be preferable when relating CL to other integral values, such as the number of neutrophils present in the volume of blood tested.

For comparison, regression analyses of integral CL values are plotted against the type of leukocyte present for blood specimens tested as illustrated in **FIGS. 3** **A, B, C** and **D.** As depicted in **FIG. 3A****,** there is some correlation, i.e., R² = 0.6782, of lucigenin luminescence with the total leukocyte count. The majority of leukocytes in blood are phagocytes, and the majority of these phagocytes are neutrophils. The correlations of lucigenin CL with the phagocyte and neutrophil counts are good, i.e., R²' s of 0.8336 and 0.8336, respectively. Lucigenin CL shows no correlation with the lymphocyte count, i.e., R² =0.0053.

Luminol CL is the product of simple (non-reductive) dioxygenation activity. With regard to phagocytes, luminol measures oxidase-driven MPO activities or eosinophil peroxidase activities. Lucigenin measures oxidase-dependent phagocyte reductive dioxygenation activity, and its CL activity is haloperoxidase independent. Measurement of phagocyte oxidase activity as lucigenin CL has advantage over luminol CL when the goal is quantifying the number of phagocytes present in blood or body fluids. Specific phagocyte NADPH oxidase activity, i.e., oxidase activity per phagocyte/neutrophil, is relatively constant and independent of variation in the MPO concentration per neutrophil. The MPO/neutrophil varies relative to the number of mitotic divisions in the myelocytic phase of myelopoiesis. Inflammatory or therapeutic increase in granulocyte colony forming factor (G-CSF) expands the promyelocytic pool where MPO is synthesized, and decreases the mitotic activity of the myelocytic pool. Each myelocyte division deletes the MPO per neutrophil by half. As such, stimulation and expansion of the promyelocytic pool and decreasing the number of divisions in the myelocytic pool result in larger neutrophils with increased MPO (Allen, Stevens et al. 1997).

Activated phagocyte NADPH oxidase activity is responsible for reductive dioxygenation activity and is measured as lucigenin CL activity. This same oxidase activity drives haloperoxidase-dependent simple (non-reductive) dioxygenation activities. Phagocyte luminol-dependent CL activity reflects haloperoxidase, especially MPO, activity. The graphics and regression analyses of **FIGS. 4** **A, B, C** and **D** show that unlike neutrophil specific oxidase activity, that correlates with the neutrophil count, luminol CL that is MPO dependent is more variable. The luminol CL per neutrophil varies with the state of host inflammation and with G-CSF or GM-CSF treatment (Allen, Stevens et al. 1997). The difference between simple (nonreducing) dioxygenation activity and reductive dioxygenation can be appreciated by comparing the integral luminol CL regression analyses of **FIGS. 4A-D**to the integral lucigenin CL regression analyses of **FIGS. 3A-D.** Note that reductive dioxygenation (lucigenin CL) plotted against neutrophils shows greater correlation with phagocytes/neutrophils, i.e., R² = 0.8336, than simple (non-reductive) dioxygenation (luminol CL) plotted against the neutrophil present R² = 0.7282. As with lucigenin CL, there is no correlation of luminol CL with the lymphocyte count, i.e., R² =0.0020.

In **FIGS. 4B** and **C**, note that the two Subjects with the highest phagocyte/neutrophil counts (two uppermost right graph points) also show high specific oxidase-driven MPO activities. High neutrophil counts and high specific MPO activities suggest G-CSF-stimulated myelopoiesis usually in response to immune-physiologic stimulation or therapeutic intervention (Allen, Stevens et al. 1997).

As illustrated in **FIGS. 5** **A** and **B,** the plots of integral luminol and lucigenin CL per neutrophil (i.e., specific activity/neutrophil) versus the age of the blood post-venipuncture show that the CL activity per neutrophil remains relatively constant during the initial post-venipuncture 16-hour interval using either luminol or lucigenin as the chemiluminigenic probe. After this initial period, neutrophil oxidase and oxidase-driven MPO activities decrease exponentially. As previously described, luminol CL activity shows more variance than lucigenin CL activity without regard to post-venipuncture age. The R²'s for the composite luminol and lucigenin CL activities per neutrophil measurements are 0.3434 and 0.6005, respectively. The lower R² observed for luminol CL is consistent with the previously described variation in MPO per neutrophil.

The post-venipuncture functional lifetime is essentially the same whether neutrophil oxidase or oxidase-driven myeloperoxidase activity is measured. As illustrated in **FIGS. 6A** and **B** for the individual Subjects 036 and 037, both oxidase-driven MPO activity per neutrophil, i.e., luminol CL, and the oxidase activity per neutrophil, i.e., lucigenin CL, show exponential decreases relative to post-venipuncture age of the blood neutrophil. The function of the hexose monophosphate shunt enzymes that provide the reducing equivalents driving both NADPH oxidase and NADPH oxidase-driven MPO activities are susceptible to age-related loss of function.

When the exponential relationships for each individual Subjects are averaged together, the relationship of luminol CL to post-venipuncture age for 20 Subjects with four complete measurements out to 60 hours was y = 59780e^{-0.026X} with a R² ± StdDev of = 0.9426 ± 0.0619. The relationship of lucigenin CL to post-venipuncture age for 24 Subjects with four complete measurements out to 60 hours was y = 55404e^{-0.024X} with a R² ± StdDev of = 0.9038 ± 0.0938.

In conclusion, chemiluminigenic probing of phagocyte and especially neutrophil NADPH oxidase activity can be applied to measuring neutrophils in blood and body fluids. Oxidase function per neutrophil is best measured using lucigenin (DBSS: N,N'-dimethyl-9,9'-biacridinium dinitrate (lucigenin) balanced salt solution) as the chemiluminigenic probe. Such lucigenin CL correlates with the number of phagocytes/neutrophils in the sub-microliter quantity of whole blood or body fluid tested. The functional activity of phagocytes in EDTA anticoagulated blood is relatively well maintained during the initial 16-hour post-venipuncture period. After this initial period, neutrophil functional capacity, measured as either oxidase activity using lucigenin CL or oxidase-driven MPO activity using luminol CL, decreases exponentially with respect to post-venipuncture age.

Luminol CL measures NADPH oxidase-driven MPO activity per neutrophil. The MPO content per neutrophil is variable and is dependent on the state of myelopoietic stimulation. Cell size, azurophilic granule content and MPO per neutrophil increase following immunologic generation of or therapeutic treatment with G-CSF (Allen, Stevens et al. 1997, Allen, Dale et al. 2000). Consequently, the ratio of oxidase-driven MPO (luminol CL) activity to oxidase (lucigenin CL) activity provides useful information with regard to treatment or immune-physiologic stimulation of neutrophil myelopoiesis.

### Definitions

As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, unless the context clearly dictates otherwise. Thus, the references "a," "an" and "the" are generally inclusive of the plurals of the respective terms. For example, reference to "a stimulus" or "the stimulus" includes a plurality of such stimuli. The term "and/or" used in the context of "X and/or Y" should be interpreted as "X," or "Y," or "X and Y." Similarly, "at least one of X or Y" should be interpreted as "X," or "Y," or "both X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of" and "consisting of" the disclosed components.

Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

"Animal" includes, but is not limited to, mammals, which includes but is not limited to rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage. As used herein, the term "patient" is understood to include an animal, for example a mammal, and preferably a human that is receiving or intended to receive treatment, as treatment is herein defined. While the terms "individual" and "patient" are often used herein to refer to a human, the present disclosure is not so limited.

### REFERENCES:

Albrecht, H. O. (1928). "Über die chemiluminescenz des aminophthalsäurehydrazids." Zeitschrift für Physikalische Chemie. **136U**: 321-330.
Allen, R. C. (1975). "Halide dependence of the myeloperoxidase-mediated antimicrobial system of the polymorphonuclear leukocyte in the phenomenon of electronic excitation." Biochemical and biophysical research communications **63**(3): 675-683.
Allen, R. C. (1975). "The role of pH in the chemiluminescent response of the myeloperoxidase-halide-HOOH antimicrobial system." Biochemical and biophysical research communications **63**(3): 684-691.
Allen, R. C. (1979). "Reduced, radical, and excited state oxygen in leukocyte microbicidal activity." Frontiers of biology **48**: 197.
Allen, R. C. (1981). "Lucigenin chemiluminescence: a new approach to the study of polymorphonuclear leukocyte redox activity." Bioluminescence and chemiluminescence: 63-73.
Allen, R. C. (1982). Biochemiexcitation: Chemiluminescence and the study of biological oxygenation. Chemical and biological generation of excited states, Academic Press New York: 310-344.
Allen, R. C. (1986). "Phagocytic leukocyte oxygenation activities and chemiluminescence: a kinetic approach to analysis." Methods in enzymology 133: 449-493.
Allen, R. C. (2019). Essence of Reducing Equivalent Transfer Powering Neutrophil Oxidative Microbicidal Action and Chemiluminescence. Neutrophils. M. Khajah. London, UK, IntechOpen: 61-85.
Allen, R. C., D. C. Dale and F. B. Taylor (2000). "Blood phagocyte luminescence: gauging systemic immune activation." Methods in enzymology 305: 591-629.
Allen, R. C. and L. D. Loose (1976). "Phagocytic activation of a luminol-dependent chemiluminescence in rabbit alveolar and peritoneal macrophages." Biochemical and biophysical research communications 69(1): 245-252.
Allen, R. C., P. R. Stevens, T. H. Price, G. S. Chatta and D. C. Dale (1997). "In vivo effects of recombinant human granulocyte colony-stimulating factor on neutrophil oxidative functions in normal human volunteers." J Infect Dis 175(5): 1184-1192.
Allen, R. C., S. J. Yevich, R. W. Orth and R. H. Steele (1974). "The superoxide anion and singlet molecular oxygen: their role in the microbicidal activity of the polymorphonuclear leukocyte." Biochemical and biophysical research communications 60(3): 909-917.
Asha, S. E., A. C. F. Chan, E. Walter, P. J. Kelly, R. L. Morton, A. Ajami, R. D. Wilson and D. Honneyman (2013). "Impact from point-of-care devices on emergency department patient processing times compared with central laboratory testing of blood samples: a randomized controlled trial and cost-effectiveness analysis." Emergency Medicine Journal 31(9): 714-719.
Bainton, D. F., Ed. (1999). Developmental biology of neutrophils and eosinophils. Inflammation, Basic Principles and Clinical Correlates. Philadelphia, Lippincott Williams & Wilkins.
Merrill, G. A., R. Bretthauer, J. Wright-Hicks and R. C. Allen (1996). "Oxygenation activities of chicken polymorphonuclear leukocytes investigated by selective chemiluminigenic probes." Laboratory Animal Science 46(5): 530-538.
POCT, A. i. "Abbott Point-of Care iSTAT Hematology."
Rossi, F., D. Romeo and P. Patriarca (1972). "Mechanism of phagocytosis-associated oxidative metabolism in polymorphonuclear leukocytes and macrophages." Journal of the Reticuloendothelial Society 12: 127.
Sbarra, A. J. and M. L. Karnovsky (1959). "The biochemical basis of phagocytosis. I. Metabolic changes during the ingesting of particles by polymorphonuclear leukocytes." Journal of Biological Chemistry 234(4): 1355-1362.
Schilling, U. M. (2014). "Time is money: the economic impact of point of care on the emergency department of a tertiary care university hospital." Point Care 13(1): 21-23.
Totter, J. R. (1964). "The quantum yield of the chemiluminescence of dimethylbiacridinium nitrate and the mechanism of its enzymatically induced chemiluminescence." Photochemistry and Photobiology 3: 231-241.

## Claims

1. A method of assessing myelopoietic suppression, or neutrophil consumption in response to infection, in a sample of whole blood of an animal, the method comprising:
diluting a sample of whole blood which has been obtained from the animal 1 to 1200 with diluting medium;
contacting the sample of diluted whole blood with:
(i) a substance that activates NADPH oxidase-dependent respiratory burst metabolism of the neutrophils present in the sample of whole blood; and
(ii) a chemiluminigenic substrate susceptible to reductive dioxygenation by the activated NADPH oxidase to yield chemiluminescence, wherein the chemiluminigenic substrate is lucigenin (N,N'-dimethyl-9,9'-biacridium dinitrate); and
quantifying the resulting reductive dioxygenation of lucigenin by measuring the emitted chemiluminescence of lucigenin using a hand-held or portable luminometer, wherein the amount of chemiluminescence is a measurement of absolute neutrophil function of the neutrophils present in the sample,
wherein the absolute neutrophil function is proportional to the absolute neutrophil count, and
wherein the absolute neutrophil count is used to assess myelopoietic suppression, or neutrophil consumption in response to infection, in the animal.

2. The method of claim 1, wherein the substance that activates NADPH oxidase-dependent respiratory burst metabolism of the neutrophils is in a solution or coated to a surface contacted by the neutrophils.

3. The method of claim 2, wherein the substance is phorbol myristate acetate.

4. The method of claim 1, further comprising adding a lectin to the sample of whole blood, wherein the lectin aggregates or removes erythrocytes from the sample of whole blood, wherein aggregation or removal of erythrocytes from the sample of whole blood facilitates chemiluminescence detection of the neutrophils present in the sample of whole blood.

5. The method of claim 1, wherein the diluted sample of whole blood is added to a microwell plate coated with the substance that activates NADPH oxidase-dependent respiratory burst metabolism of the neutrophils present in the sample of whole blood and containing a solution of lucigenin.

## Patentansprüche

1. Verfahren zum Beurteilen der myelopoietischen Suppression oder des Neutrophilenverbrauchs als Reaktion auf eine Infektion in einer Vollblutprobe eines Tieres, das Verfahren umfassend:
Verdünnen einer dem Tier entnommenen Vollblutprobe 1 zu 1.200 mit Verdünnungsmedium;
Inkontaktbringen der verdünntem Vollblutprobe mit:
(i) einer Substanz, die den NADPH-Oxidase-abhängigen respiratorischen Burst-Stoffwechsel der in der Vollblutprobe vorhandenen Neutrophilen aktiviert; und
(ii) einem chemiluminogenen Substrat, das für reduktive Dioxygenierung durch die aktivierte NADPH-Oxidase empfänglich ist, um Chemilumineszenz zu ergeben, wobei das chemiluminogene Substrat Lucigenin (N,N'-Dimethyl-9,9'-biacridiumdinitrat) ist; und
Quantifizieren der resultierenden reduktiven Dioxygenierung von Lucigenin durch Messen der emittierten Chemilumineszenz von Lucigenin unter Verwendung eines handgehaltenen oder tragbaren Luminometers, wobei der Betrag der Chemilumineszenz ein Maß der absoluten Neutrophilenfunktion der in der Probe vorhandenen Neutrophilen ist,
wobei die absolute Neutrophilenfunktion proportional zu der absoluten Neutrophilenzahl ist, und
wobei die absolute Neutrophilenzahl verwendet wird, um die myelopoietische Suppression oder den Neutrophilenverbrauch in der Reaktion auf eine Infektion in dem Tier zu bewerten.

2. Verfahren nach Anspruch 1, wobei die Substanz, die den NADPH-Oxidase-abhängigen respiratorischen Burst-Stoffwechsel der Neutrophilen aktiviert, in einer Lösung vorliegt oder auf eine von den Neutrophilen kontaktierte Oberfläche aufgetragen ist.

3. Verfahren nach Anspruch 2, wobei die Substanz Phorbolmyristatacetat ist.

4. Verfahren nach Anspruch 1, ferner umfassend Zugeben eines Lektins zur Vollblutprobe, wobei das Lektin Erythrozyten aus der Vollblutprobe aggregiert oder entfernt, wobei das Aggregieren oder Entfernen von Erythrozyten aus der Vollblutprobe den Chemilumineszenz-Nachweis der in der Vollblutprobe vorhandenen Neutrophilen erleichtert.

5. Verfahren nach Anspruch 1, wobei die verdünnte Vollblutprobe zu einer Mikrotiterplatte gegeben wird, die mit der Substanz beschichtet ist, die den NADPH-Oxidase-abhängigen respiratorischen Burst-Stoffwechsel der in der Vollblutprobe vorhandenen Neutrophilen aktiviert, und die eine Lösung von Lucigenin enthält.

## Revendications

1. Procédé d'évaluation de la suppression myélopoïétique, ou de la consommation de neutrophiles en réponse à une infection, dans un échantillon de sang total d'un animal, le procédé comprenant :
la dilution, d'un échantillon de sang total qui a été obtenu à partir de l'animal, 1 pour 1200 avec un milieu de dilution ;
la mise en contact de l'échantillon de sang total dilué avec :
(i) une substance qui active le métabolisme de stimulation du métabolisme oxydatif dépendant de la NADPH oxydase des neutrophiles présents dans l'échantillon de sang total ; et
(ii) un substrat chimioluminigène susceptible d'une dioxygénation réductrice par la NADPH oxydase activée pour produire une chimiluminescence, dans lequel le substrat chimioluminigène est la lucigénine (N,N'-diméthyl-9,9'-biacridium dinitrate) ; et
la quantification de la dioxygénation réductrice résultante de la lucigénine en mesurant la chimiluminescence émise de la lucigénine à l'aide d'un luminomètre portable ou tenu à la main,
dans lequel la quantité de chimiluminescence est une mesure de la fonction neutrophile absolue des neutrophiles présents dans l'échantillon,
dans lequel la fonction neutrophile absolue est proportionnelle au nombre absolu de polynucléaires neutrophiles, et
dans lequel le nombre absolu de polynucléaires neutrophiles est utilisé pour évaluer la suppression myélopoïétique, ou la consommation de neutrophiles en réponse à une infection, chez l'animal.

2. Procédé selon la revendication 1, dans lequel la substance qui active le métabolisme de stimulation du métabolisme oxydatif dépendant de la NADPH oxydase des neutrophiles est dans une solution ou est enrobée sur une surface en contact avec les neutrophiles.

3. Procédé selon la revendication 2, dans lequel la substance est le phorbol myristate acétate.

4. Procédé selon la revendication 1, comprenant en outre l'ajout d'une lectine à l'échantillon de sang total, dans lequel la lectine agrège ou élimine les érythrocytes issus de l'échantillon de sang total, dans lequel l'agrégation ou l'élimination des érythrocytes issus de l'échantillon de sang total facilite la détection par chimiluminescence des neutrophiles présents dans l'échantillon de sang total.

5. Procédé selon la revendication 1, dans lequel l'échantillon dilué de sang total est ajouté à une plaque de microcupules enrobées de la substance qui active le métabolisme de stimulation du métabolisme oxydatif dépendant de la NADPH oxydase des neutrophiles présents dans l'échantillon de sang total et contenant une solution de lucigénine.
